# EUROPEAN PATENT SPECIFICATION

(11) **EP 0 975 619 B1**
(45) Date of publication and mention of the grant of the patent: **03.01.2007**
(21) Application number: 98913903.5
(22) Date of filing: 25.03.1998
(51) Int. Cl.: C07D 311/92, C07D 311/78, C07D 409/04, G02B 5/23

(54) **INTENSE COLOURING PHOTOCHROMIC 2H-NAPHTHO[1,2-b] PYRANS AND HETEROCYCLIC PYRANS**
STARK FÄRBENDE PHOTOCHROME 2H-NAPHTHO[1,2-b])PYRANE UND HETEROCYCLISCHE PYRANE
2H-NAPHTO[1,2-b]PYRANS PHOTOCHROMIQUES A COLORATION INTENSE ET PYRANS HETEROCYCLIQUES

(30) Priority: 25.03.1997 GB 9706203
(43) Date of publication of application: 02.02.2000
(73) Proprietor: JAMES ROBINSON LIMITED, Huddersfield, West Yorkshire HD1 6BU (GB)
(72) Inventor: CLARKE, David, Allan, Rastrick Brighouse HD6 3XD (GB); HERON, Bernard, Mark, East Riding Yorkshire HU15 1AB (GB); GABBUTT, Christopher, David, Preston Lancashire PR3 2YS (GB); HEPWORTH, John, David, Preston Lancashire PR2 7ER (GB); PARTINGTON, Steven, Michael, Golcar Huddersfield HD7 4PG (GB); CORNS, Stephen, Nigel, Paddock Huddersfield HD1 4JN (GB)
(74) Representative: Wain, Christopher Paul
(86) International application number: PCT/GB1998/000905
(87) International publication number: WO 1998/042695

(56) References cited:
- EP-A- 0 250 193
- WO-A-95/16215
- WO-A-96/04576
- WO-A-98/04937
- US-A- 5 552 091
- US-A- 5 658 500

## Description

The present invention relates to certain new photochromic pyran derivatives and to their use.

Photochromism is a well-known physical phenomenon which is observed with certain classes of chemical compounds. A detailed discussion of this phenomenon can be found in "Photochromism: Molecules and Systems," Studies in Organic Chemistry 40, Eds. H Dürr and H. Bouas-Laurent, Elsevier, 1990.

The 2*H*-naphtho[1,2-*b*]pyran system is known to be capable of exerting a photochromic effect as described, for example, U. S. Patent No. 3,567,605 and U. S. Patent No. 4,826,977. U. S. Patent No. 3,567,605 provides an example of a 2*H*-naphtho[1,2-*b*]pyran which remains coloured at ambient temperatures for several hours, and U. S. Patent No. 4,826,977 describes a series of yellow/orange colouring 2*H*-naphtho[1,2-*b*]pyrans containing a spiro-adamantane group at the 2-position, amongst other 2*H*-[1]benzopyran and isomeric naphthopyran systems. The basic structural unit of the 2*H*-naphtho[1,2-*b*]pyran system, in this instance substituted at C-2 with a spiro-adamantane group, is illustrated below.

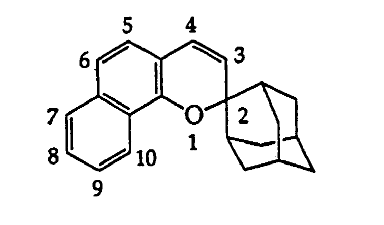

A range of purple/blue colouring 2(4-aminophenyl)-2-alkyl-2H-naphtho[1,2-*b*]pyrans have been described in U. S. Patent No. 4,818,096 and European Patent No. 0,250,193 describes a range of photochromic naphtho-[1,2-*b*] and [2,1-*b*]pyrans which bear one or two aminophenyl substituents on the carbon atom adjacent to the oxygen heteroatom. In this patent it is stated that substitution in the ring positions, sites 5 -10, other than at site 6 has little influence on the photochromic behaviour of the compounds.

A series of photochromic 2*H*-naphtho[1,2-*b*]pyrans, amongst other 2*H*-[1]benzopyrans and isomeric naphthopyrans, bearing a cyclopropyl group as one of the substituents at the 2-position is described in article WO92/01959. It is also commented that the compound 2-cyclopropyl-2-*p-*methoxyphenyl-5-methyl-2*H*-naphtho[1,2-*b*]pyran and several other analogues are of particular current interest, but no reasons were presented either to substantiate such interest or as to any significance of the 5-methyl group.

It is stated in U. S. Patent No. 5,066,818 (1991) that "The compound, 2,2-diphenyl-2*H*-naphtho[1,2-*b*]pyran, also colours on exposure to near ultraviolet light at room temperature but does not bleach in a reasonable period of time. Substitution of the phenyl substituents in the *meta* and *para* positions have little effect on the rate of bleaching of these compounds."

The very high optical density of 2,2-diaryl-2*H*-naphtho[1,2-*b*]pyrans achieved under irradiation and their slow attendant fade (bleaching) on removal of the source of irradiation relative to the photochromic properties displayed by the isomeric 3,3-diaryl-3*H*-naphtho[2,1-*b*]pyrans has been recently noted by B. van Gemert *et al.* (*Mol. Cryst. Liq. Cryst.,* 1994, 246, 67). The relatively slow attendant fade of the 2,2-diaryl-2*H*-naphtho[1,2-*b*]pyrans was rationalised by the absence of steric crowding in the ring opened (coloured) quinoidal/zwitterionic forms. Such steric crowding is thought to be present for the ring opened form of the 3,3-diaryl-3*H*-naphtho[2,1-*b*]pyrans and accounts for their relatively rapid fade.

Pilkington Brothers Limited have also commented on the fading of photochromic materials in Research Disclosure. Two structurally similar deep colouring photochromic 2,2-diaryl-2*H*-naphtho[1,2-*b*]pyrans, namely 2,2-bis(4-methoxyphenyl)-5,6-dimethyl-2*H*-naphtho[1,2-*b*] pyran and 2-(4-methoxyphenyl)-2-(4-trifluoromethylphenyl)-5,6-dimethyl-2*H*-naphtho-[1,2-*b*]pyran are described, which exhibit markedly improved attendant fade compared with the non-methyl substituted analogues. These improved rates of fade are attributed to the combined presence of methyl groups at the 5- and 6-positions, which are said to exert steric pressures upon the ring opened (coloured) quinoidal/zwitterionic forms, thereby enhancing the ring closure to the uncoloured naphthopyran system. However, these fast fade materials described by Pilkington plc with substituents at both the 5- and 6- positions are difficult to make, requiring a long multi-stage process which renders them unattractive commercially. Thus the use of two substituents at the 5- and 6-positions to achieve rapid fade in these 2,2-diaryl compounds has the disadvantage of manufacture complexities.

Two recent U. S. Patents, 5,458,814 and 5,514,817 describe the synthesis of a range fast fading intensly colouring 5-substituted or 5,6-disubstituted 2,2-diaryl-2*H*-naphtho[1,2-*b*]pyrans and phenanthropyrans.

US patent no. 5,552,091 describes photochromic benzopyrans the general formula of which embraces compounds substituted at the 2-position of the pyran ring by one 4-aminophenyl or -naphthyl group. However, the other 2-position of the pyran ring is substituted with a dibenzo-fused 5-member heterocyclic compound, and there is no substitution at the 5-position.

We have investigated these known photochromic compounds and have found that, for enhanced intense colour generation, compounds having 2-(aminoaryl)-2-aryl or 2,2-bis(aminoaryl) substituents are preferred. Also the presence of a 5-substituent in these 2,2-diaryl-2*H*-naphtho[1,2-*b*]pyrans ensures rapid fading of the red or orange colour generated upon irradiation.

According to the present invention, there is provided a naphtho[1,2-b]pyran of general formula 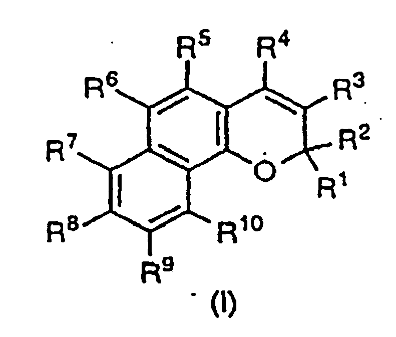 wherein at least one of R¹ and R² is a 4-aminoarylgroup, wherein the aryl group is phenyl or naphthyl, and wherein the amino group which forms part of the 4-aminoaryl group for R¹ and R² is C₁-C₅ alkylamino, C₁-C₅ dialkylamino, arylamino, diarylamino, aryl C₁-C₅ alkylamino, or a cyclic amino group, wherein the 4-aminoaryl group may optionally be further substituted in addition to the specified amino function, and in any remaining positions with hydrogen, C₁-C₅ alkyl, C₁-C₅ haloalkyl, C₁-C₅ alkoxy, C₁-C₅- alkoxy(C₁-C₅)alkyl, C₁-C₅ hydroxy alkyl, C₁-C₅ amino alkyl, halogen, alkyl C₁-C₅ amino, dialkyl C₁-C₅ amino, arylamino, diarylamino, or a cyclic amino group; and wherein where R¹ or R² is not a 4-aminoaryl group it is selected from phenyl, substituted phenyl, naphthyl, substituted naphthyl, thienyl, benzo[b]thienyl, furyl, benzo[b]furyl, pyrryl or indolyl;
R⁵ is selected from linear or branched C₁-C₁₀ alkyl, up to C₂₀ cycloalkyl, up to C₂₀ bicycloalkyl, up to C₂₀ polycycloalkyl, linear or branched up to C₁₀ alkenyl, up to C₁₀ alkynyl, linear or branched C₁-C₁₀ alkoxy, linear or branched C₁-C₁₀ alkylthio, linear or branched C₁-C₁₀ alkoxy (linear or branched C₁-C₁₀)alkyl, linear or branched C₁-C₁₀ hydroxyalkyl, linear or branched C₁-C₁₀ aminoalkyl, phenyl, halogen, nitrile, nitro, linear or branched C₁-C₂₀ alkoxycarbonyl, formyl, acetyl, aroyl, or is the alkenyl function : 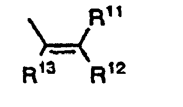 wherein R¹¹ and/or R¹² and/or R¹³ is hydrogen or is as defined for R⁵ above excluding the alkenyl function; and R³ and R⁴ are hydrogen; R⁶-R¹⁰ are each hydrogen or as defined for R¹, R² or R⁵ above.

Phenyl ring substituents may be located at the *o*-, *m-* or *p*-positions. Typically each phenyl group contains less than 3 substituents.

Typically, though not always, two or three groups selected from R⁷, R⁸, R⁹ and R¹⁰ are hydrogen.

In addition to the 2*H*-naphtho[1,2-b]pyran compounds of formula I, the present invention includes the isomeric phenanthropyrans of the general formula II and III and benzo[1] phenanthropyrans of the general formula IV:

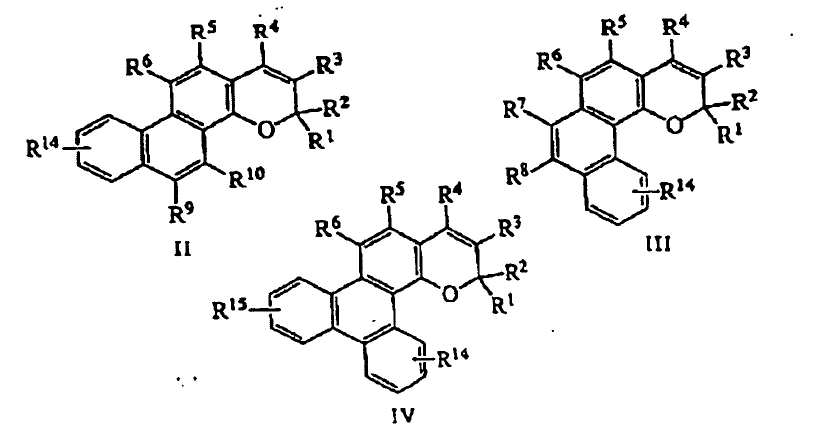

In graphic formula II, III and IV, R¹ to R¹³ inclusive are as specified for graphic formula I and R¹⁴ and R¹⁵ may be selected from those substituents specified for R⁷ to R¹⁰ inclusive.

In addition to the 2*H*-naphtho[1,2-*b*]pyran compounds of formula I, the present invention includes the isomeric heterocyclicpyrans of the general formula V and VI:

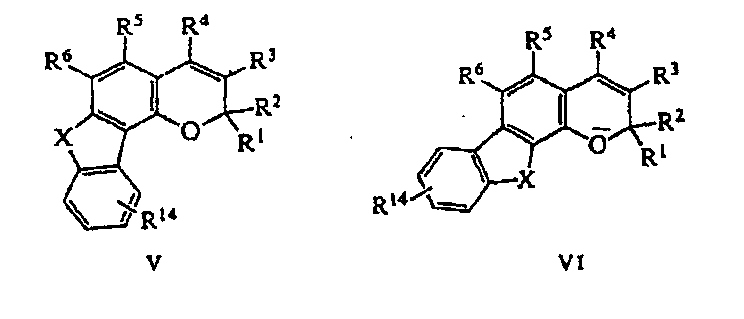

In graphic formula V and VI, R¹ to R¹⁴ are as specified above and the heteroatom X may be selected from O, S, NH and substituted N for example C₁ - C₁₀ alkyl, C₁ - C₁₀ haloalkyl, C₁-C₁₀ perfluoroalkyl, benzyl, phenyl, tosyl, benzoyl, amino-C₁ - C₅ alkyl, hydroxy-C₁ - C₅ alkyl.

The photochromic properties exhibited by the novel pyran compounds of the present invention, namely those of high induced optical density and rapid bleaching of the red or orange coloured form, render these compounds particularly useful as photochromic materials for incorporation into polymeric host materials so as to impart photochromic properties to the said polymeric host materials. Examples of applications of the polymeric host materials containing photochromic materials of the present invention include the manufacture of lenses for sunglasses and ophthalmic lenses, optical filters and windows for vehicles such as cars (including sunroofs), aircraft and ships and architectural uses e.g. windows for homes and for photochromic 'stained glass' windows.

The photochromic pyrans of the present invention are incorporated into the 'plastic' host material by well established protocols for example as described in European Patent No. 0254020 or U. S. Patent No. 5,066,818.

The high induced optical density of the photochromic compounds of the present invention enables the amount of the photochromic material required so as to impart a useful degree of photochromism to a polymeric host material or to a solution to be greatly reduced, thereby enabling a considerable saving of synthetic effort and cost. Furthermore, the use of reduced quantities of the photochromic materials of the present invention has the bonus that there is a consequent reduction in any undesirable colour that the photochromic materials may impart in the bleached state, either by way of inherent colour of the material itself or by the formation of coloured fatigue / degradation products through use of the photochromic material.

Typical host materials are optically clear polymer materials, such as polymers of polyol (allyl carbonate) - monomers, polyacrylates such as polymethylmethacrylates, cellulose acetate, cellulose triacetate, cellulose acetate propionate, cellulose acetate butyrate, poly(vinyl acetate), poly(vinyl alcohol), polyurethanes, polycarbonate, polyethylene terephthalate, polystyrene, poly(triethyleneglycol dimethylacrylate), poly(diethyleneglycol bis(allyl carbonate)) and various copolymer mixes.

The pyran compounds of the present invention may be prepared by a general method which is based on the following reaction scheme:

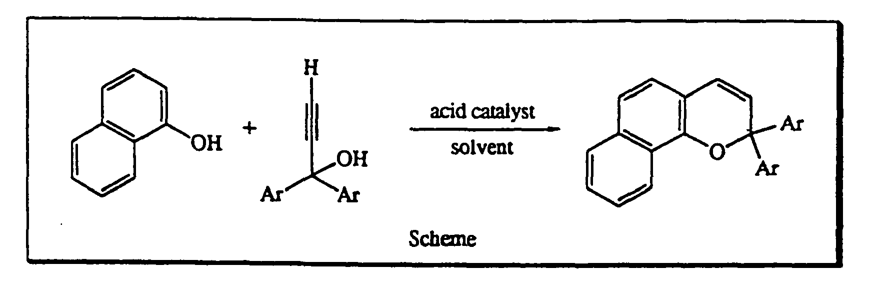

This general synthetic methodology has been descibed in detail, for example, by L. Merlini in 'Advances in Heterocyclic Chemistry; 1975, vol. 18, page 159, and by R. Guglielmetti in "Photochromism: Molecules and Systems," Studies in Organic Chemistry 40, chp. 8, Eds. H Dürr and H. Bouas-Laurent, Elsevier, 1990, and also in several patent documents, for example, U. S. Patent No. 5,066,818; U. S. Patent No. 4,990,287, WO 92/09593 and WO95/05382. The synthesis of the propargyl alcohols shown in the scheme above are obtained in a known manner, for example, T. F. Rutledge in 'Acetylenic Compounds,' Reinhold, New York, 1968. The 1-naphthols and related hydroxy compounds are either commercially available or obtained by known synthetic methods, or derived from such methods. Some of the 1-naphthols and related hydroxy compounds or precursors thereof have been described in the chemical literature, for example, ethyl 1-acetoxydibenzo thiophene-3-carboxylate see (S. Gronowitz *et al.,* Acta. Pharm. Suec., 1978, **15,** 337) and 3-hydroxypropyl-1-naphthol see (R. F. Frank *et al.,* J. Chem. Soc., Chem. Commun., 1984, 761). The use of the Stobbe condensation to prepare 1-naphthols has also been discussed (see Organic Reactions 1951, 6, 1). The acid catalyst may be selected from acidic alumina (Brockmann 1), acetic acid, trifluoroacetic acid, silica, clays (e.g. montmorillionite, tonsil) or acidic exchange resins.
Organic solvents frequently employed for the reaction include benzene, toluene, xylene and relatively high boiling alkanes.

The following examples illustrate but do not limit the invention:

### Example 1: Methyl 9-methoxy-2-phenyl-2-(2-thienyl)-2H-naphtho[1,2-b]pyran-5-carboxylate

### (a) Ethyl 4-acetoxy-6-methoxy-2-raphthoate

A solution of freshly distilled p-anisaldehyde (20g, 146.9 mmol) and diethyl succinate (38.4g, 220.3 mmol) in anhydrous ethanol (50 cm³) was added dropwise over 45 minutes to a vigorously stirred warm ∼ 40 - 50 °C, solution of sodium ethoxide (from sodium 6.75g, 293.8 mmol) in anhydrous ethanol (450 cm³) under N₂. On completion of the addition the solution was refluxed for 4 hours and then cooled to room temperature.

The reaction mixture was reduced to ∼ 1/5 of the original volume and the resulting viscous oil was diluted with water (700 cm³), cautiously acidified with c. HCl and the resulting two phase mixture extracted with ethyl acetate (5 x 100 cm³). The combined EtOAc solutions were extracted with aq. sat. NaHCO₃ solution (6 x 100 cm³). The combined aq. NaHCO₃ solutions were cautiously acidified with c. HCl and the resulting two phase mixture extracted with EtOAc (4 x 100 cm³). The combined EtOAc extracts were dried (Na₂SO₄) and evaporated to afford a yellow mobile oil.

A solution of the aforegoing yellow oil and anhydrous sodium acetate (12.05g, 146.9 mmol) in acetic anhydride (180 cm³) was refluxed for 3 hours. The solution was cooled to room temperature and then diluted with water (2000 cm³) and allowed to stir for 1.5 hours. The resulting pale brown solid was collected by vacuum filtration, washed well with water (~500 cm³) and air dried.

The solid was recrystallised from EtOAc / hexane and Norit (activated charcoal) to give ethyl 4-acetoxy-6-methoxy-2-naphthoate (yield = 21.2 g, theoretical yield = 42.35 g, 50 %, m. p. = 103.5 -104:5 °C (uncorrected)).

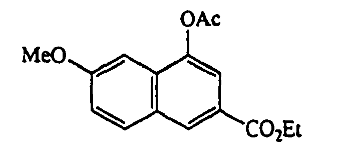

### (b) Methyl 4-hydroxy-6-methoxy-2-naphthoate

A solution of ethyl 4-acetoxy-6-methoxy-2-naphthoate (3.0g, 10.4 mmol) and sodium hydroxide (2.5g, 62.5 mmol) in water (60 cm³) and ethanol (15 cm³) was maintained at 80 - 90 °C for 3 hours. The cooled solution was poured into water (400 cm³) and cautiously acidified with c. HCl. The resulting suspension was extracted with EtOAc (5 x 75 cm³). The combined extracts were dried (Na₂SO₄) and evaporated to give a pale brown solid. This solid was dissolved in methanol (50 cm³) containing c. H₂SO₄ (∼ 1 cm³) and was refluxed for 4 hours. The cooled mixture was diluted with water (500 cm³) and extracted with EtOAc (4 x 50 cm³). The combined extracts were washed with aq. sat. NaHCO₃ (2 x 100 cm³) and water (100 cm³). Removal of the dried (Na₂SO₄) EtOAc gave a pale brown solid which was recrystallised from EtOAc/hexane to afford methyl 4-hydroxy-6-methoxy-2-naphthoate (yield = 1.63g, theoretical yield = 2.41g, 68%, m.p. = 193 - -195 °C (uncorrected)).

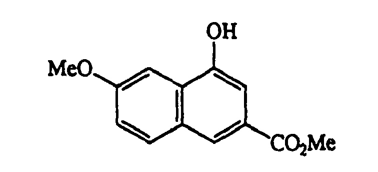

### (c) Methyl 9-methoxy-2-phenyl-2-(2-thienyl)-2H-naphtho[1,2-b]pyran-5-carboxylate

A solution of methyl 4-hydroxy-6-methoxy-2-naphthoate (0.45g, 1.8 mmol) and 1-(4-morpholinophenyl)-1-(2-thienyl)prop-2-yn-1-ol (0,55g, 1.8 mmol) in toluene (45 cm³) containing acidic alumina (Brockmann 1), (4.0g) was refluxed for 60 minutes. The cooled solution was filtered and the alumina was washed well with EtOAc (200 cm³). Removal of the solvent gave an oil which solidified on standing at RT. Recrystallisation twice from EtOAc/hexane gave methyl 9-methoxy-2-phenyl-2-(2-thienyl)-2*H-*naphtho[1,2-*b*]pyran-5-carboxylate (yield = 0.49 g, theoretical yield = 0.93g 52%, m.p. = 186 - 188°C (uncorrected)).

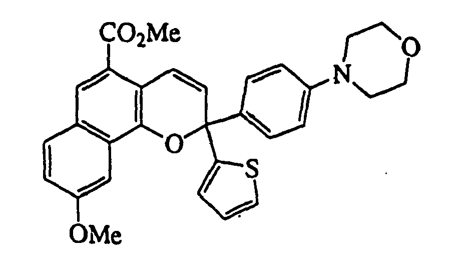

Example 2: Methyl 9-methoxy-2-(4-morpholinophenyl)-2-phenyl-2*H-*naphtho[1,2-*b*]pyran-5-carboxylate, m.p. = 175 - 177 °C (uncorrected). This compound was obtained by a similar protocol to example 1 above using the requisite starting materials.

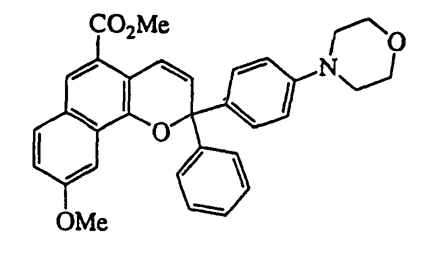

Example 3: Methyl 9-methoxy-2,2-bis(4-pyrrolidinophenyl)-2*H*-naphtho[1,2-*b*]pyran-5-carboxylate, m.p. = 210 - 215°C (uncorrected). This compound was obtained by a similar protocol to example 1 above using the requisite starting materials.

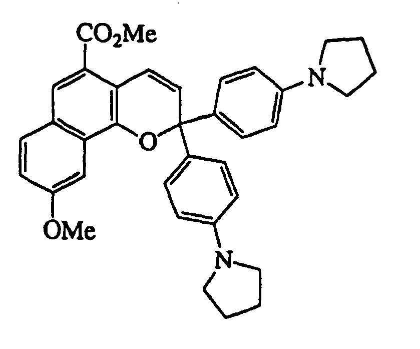

Example 4: Methyl 9-methoxy-2-phenyl-2-(4-piperidinophenyl)-2*H-*naphtho[1,2-*b*]pyran-5-carboxylate, m.p. = 164 - 167 °C (uncorrected). This compound was obtained by a similar protocol to example 1 above using the requisite starting materials.

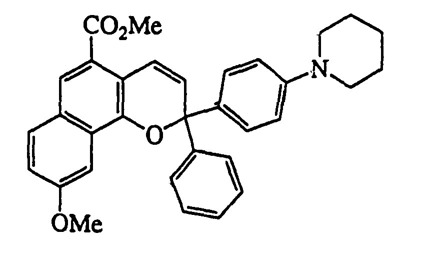

Example 5: Methyl 2-(4-methoxyphenyl)-2-(4-morpholinophenyl)-2*H-*naphtho[1,2-*b*]pyran-5-carboxylate, m.p. = 177 - 179 °C (uncorrected). This compound was obtained by a similar protocol to example 1 above using the requisite starting materials.

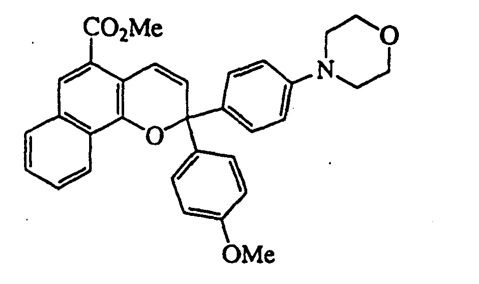

Example 6: Methyl 7,9-dichloro-2-(4-pyrrolidinophenyl)-2-phenyl-2*H-*naphtho[1,2-*b*]pyran-5-carboxylate, m.p. = 162 - 165 °C (uncorrected). This compound was obtained by a similar protocol to example 1 above using the requisite starting materials.

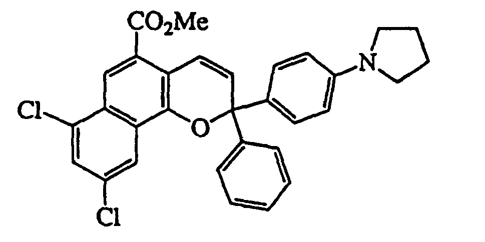

Example 7: Methyl 7-fluoro-2-(4-piperidinophenyl)-2-phenyl-2*H*-naphtho[1,2-naphtho[1,2-*b*]pyran-5-carboxylate, m.p. = 165 - 168 °C (uncorrected). This compound was obtained by a similar protocol to example 1 above using the requisite starting materials.

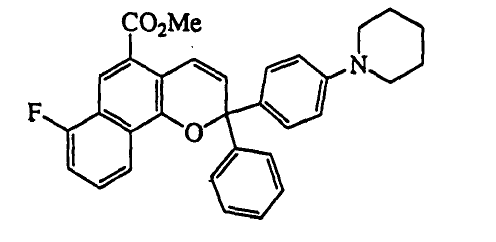

### Comparative example 1: 2-(4-morpholinophenyl)-2-phenyl-2H-naphtho[1,2-b]pyran, m.p. = 131 - 134 °C (uncorrected).

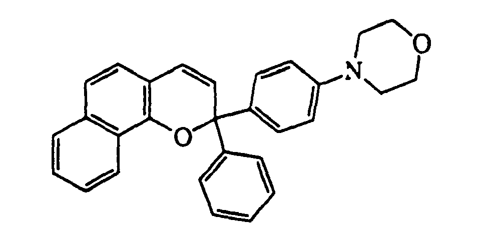

### Comparative example 2: Methyl 9-methoxy-2,2-bis(4-methoxyphenyl)-2H-naphtho[1,2-b]pyran-5-carboxylate

### (a) Ethyl 4-acetoxy-6-methoxy-2-naphthoate

A solution of freshly distilled p-anisaldehyde (20g, 146.9 mmol) and diethyl succinate (38.4g, 220.3 mmol) in anhydrous ethanol (50 cm³) was added dropwise over 45 minutes to a vigorously stirred warm ∼ 40 - 50 °C, solution of sodium ethoxide (from sodium 6.75g, 293.8 mmol) in anhydrous ethanol (450 cm³) under N₂. On completion of the addition the solution was refluxed for 4 hours and then cooled to room temperature.

The reaction mixture was reduced to ~ 1/5 of the original volume and the resulting viscous oil was diluted with water (700 cm³), cautiously acidified with c. HCl and the resulting two phase mixture extracted with ethyl acetate (5 x 100 cm³). The combined EtOAc solutions were extracted with aq. sat. NaHCO₃ solution (6 x 100 cm³). The combined aq. NaHCO₃ solutions were cautiously acidified with c. HCl and the resulting two phase mixture extracted with EtOAc (4 x 100 cm³). The combined EtOAc extracts were dried (Na₂SO₄) and evaporated to afford a yellow mobile oil.

A solution of the aforegoing yellow oil and anhydrous sodium acetate (12.05g, 146.9 mmol) in acetic anhydride (180 cm³) was refluxed for 3 hours. The solution was cooled to room temperature and then diluted with water (2000 cm³) and allowed to stir for 1.5 hours. The resulting pale brown solid was collected by vacuum filtration, washed well with water (~500 cm³) and air dried.

The solid was recrystallised from EtOAc / hexane and Norit (activated charcoal) to give ethyl 4-acetoxy-6-methoxy-2-naphthoate (yield = 21.2 g, theoretical yield = 42.35 g, 50 %, m. p. = 103.5-104.5 °C (uncorrected)).

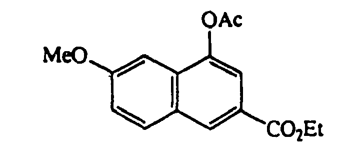

### (b) Methyl 4-hydroxy-6-methoxy-2-naphthoate

A solution of ethyl 4-acetoxy-6-methoxy-2-naphthoate (3.0g, 10.4 mmol) and sodium hydroxide (2.5g, 62.5 mmol) in water (60 cm³) and ethanol (15 cm³) was maintained at 80 - 90 °C for 3 hours. The cooled solution was poured into water (400 cm³) and cautiously acidified with c. HCl. The resulting suspension was extracted with EtOAc (5 x 75 cm³). The combined extracts were dried (Na₂SO₄) and evaporated to give a pale brown solid. This solid was dissolved in methanol (50 cm³) containing c. H₂SO₄ (~1 cm³) and was refluxed for 4 hours. The cooled mixture was diluted with water (500 cm³) and extracted with EtOAc (4 x 50 cm³). The combined extracts were washed with aq. sat. NaHCO₃ (2 x 100 cm³) and water (100 cm³). Removal of the dried (Na₂SO₄) EtOAc gave a pale brown solid which was recrystallised from EtOAc/hexane to afford methyl 4-hydroxy-6-methoxy-2-naphthoate (yield = 1.63g, theoretical yield = 2.41g, 68%, m.p. = 193 - 195 °C (uncorrected)).

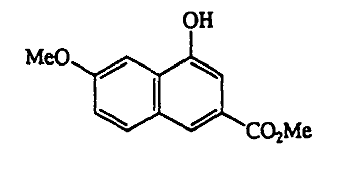

### (c) Methyl 9-methoxy-2,2-bis(4-methoxyphenyl)-2H-naphtho [1,2-b]pyran-5-carboxylate.

A solution of methyl 4-hydroxy-6-methoxy-2-naphthoate (1.0g, 4.3 mmol) and 1,1-di(4-methoxyphenyl)prop-2-yn-1-ol (1.16g, 4.3 mmol) in toluene (45 cm³) containing acidic alumina (Brockmann 1), (4.0g) was refluxed for 45 minutes. The cooled solution was filtered and the alumina was washed well with EtOAc (200 cm³). The organic filtrate was washed with aqueous sodium hydroxide (2M, 2 x 50 cm³) and water (10.0 cm³). Removal of the dried (Na₂SO₄) EtOAc gave an oil which was flash chromatographed over silica using 25% EtOAc in hexane as the eluent to afford a pale yellow solid. Recrystallisation from EtOAc/hexane gave methyl 9-methoxy-2,2-bis(4-methoxyphenyl)-2*H*-naphtho[1,2-*b*]pyran-5-carboxylate (yield = 0.79g, theoretical yield = 2.08g 38%, m.p. = 162.5 - 164.0 °C (uncorrected)).

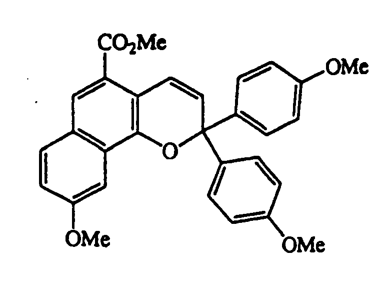

## Claims

1. A naphtho[1,2-b]pyran of general formula 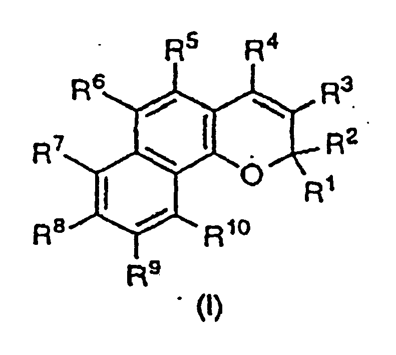 wherein at least one of R¹ and R² is a 4-aminoarylgroup, wherein the aryl group is phenyl or naphthyl, and wherein the amino group which forms part of the 4-aminoaryl group for R¹ and R² is C₁-C₅ alkylamino, C₁-C₅ dialkylamino, arylamino, diarylamino, aryl C₁-C₅ alkylamino, or a cyclic amino group, wherein the 4-aminoaryl group may optionally be further substituted in addition to the specified amino function, and in any remaining positions with hydrogen, C₁-C₅ alkyl, C₁-C₅ haloalkyl, C₁-C₅ alkoxy, C₁-C₅ alkoxy(C₁-C₅)alkyl, C₁-C₅ hydroxy alkyl, C₁-C₅ amino alkyl, halogen, alkyl C₁-C₅ amino, dialkyl C₁-C₅ amino, arylamino, diarylamino, or a cyclic amino group; and wherein where R¹ or R² is not a 4-aminoaryl group it is selected from phenyl, substituted phenyl, naphthyl, substituted naphthyl, thienyl, benzo[b]thienyl, furyl, benzo[b]furyl, pyrryl or indolyl;
R⁵ is selected from linear or branched C₁-C₁₀ alkyl, up to C₂₀ cycloalkyl, up to C₂₀ bicycloalkyl, up to C₂₀ polycycloalkyl, linear or branched up to C₁₀ alkenyl, up to C₁₀ alkynyl, linear or branched C₁-C₁₀ alkoxy, linear or branched C₁-C₁₀ alkylthio, linear or branched C₁-C₁₀ alkoxy (linear or branched C₁-C₁₀)alkyl, linear or branched C₁-C₁₀ hydroxyalkyl, linear or branched C₁-C₁₀ aminoalkyl, phenyl, halogen, nitrile, nitro, linear or branched C₁-C₂₀ alkoxycarbonyl, formyl, acetyl, aroyl, or is the alkenyl function : 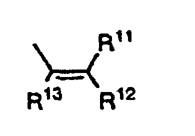 wherein R¹¹ and/or R¹² and/or R¹³ is hydrogen or is as defined for R⁵ above excluding the alkenyl function; and R³ and R⁴ are hydrogen; R⁶-R¹⁰ are each hydrogen or as defined for R¹, R² or R⁵ above.

2. A naphtho[1,2-b]pyran according to claim 1, wherein the cyclic amino group is aziridino, pyrrolidino, piperidino, morpholino, thiomorpholino, indolino, piperazino, C₁-C₅N-Alkylpiperazino or N-arylpiperazino.

3. A naphtho[1,2-b]pyran of the general formula II, III or IV: 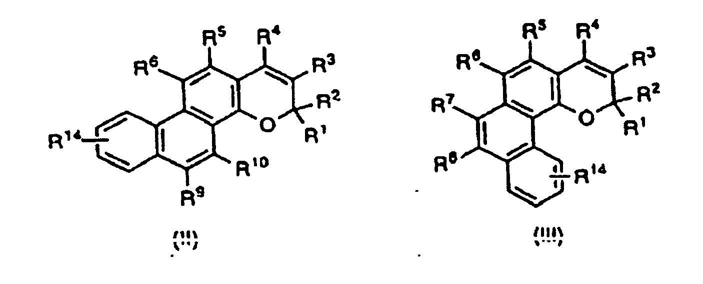 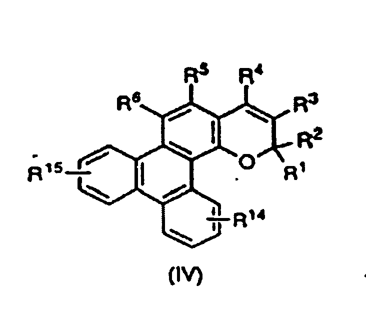 wherein R¹ to R¹³ inclusive are as defined in claim 1 or 2 and wherein R¹⁴ and R¹⁵ are as defined for R⁷-R¹⁰.

4. A naphtho[1,2-b]pyran of general formula V or VI: 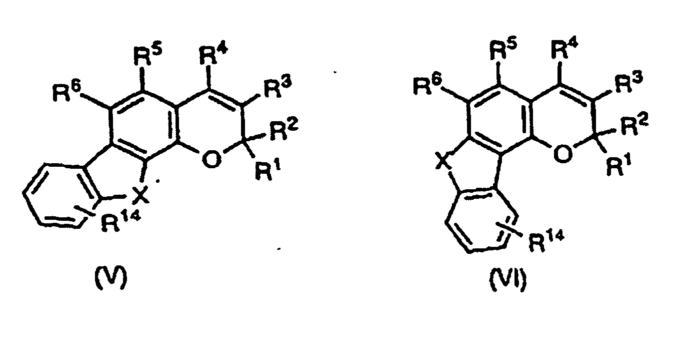 wherein R¹ to R¹⁴ inclusive are as defined in claim 3, X is selected from O, S, SO, SO₂, Se, NH, N-linear or branched C₁-C₁₀ alkyl, N-aryl, N-heteroaryl, N-linear or branched C₁-C₁₀ haloalkyl, N-linear or branched C₁-C₁₀ perhaloalkyl, N-linear or branched C₁-C₁₀ hydroxyalkyl, N-linear or branched C₁-C₁₀ alkoxyalkyl, benzyl, substituted benzyl and tosyl.

5. A naphtho[1,2-b]pyran according to any preceding claim, wherein R¹ is 4-morpholinophenyl, 4-piperidinophenyl, 4-dimethylaminophenyl or 4-pyrrolidinophenyl and R⁵ is methoxycarbonyl.

6. A naphtho[1,2-b]pyran according to any one of claims 1 to 5, wherein R¹ and R² are each 4-pyrrolidinophenyl and R⁵ is methoxycarbonyl.

7. A naphtho[1,2-b]pyran according to any one of claims 1 to 5, wherein R¹ is 4-morpholinophenyl, R² is 4-methoxyphenyl and R⁵ is methoxycarbonyl.

8. A naphtho[1,2-b]pyran according to any one of claims 1 to 5, wherein R¹ is 4-morpholinophenyl, R² is 2-thienyl and R⁵ is methoxycarbonyl.

9. A polymeric host material including a naphtho[1,2-b]pyran according to any preceding claim.

10. A polymeric host material according to claim 9, wherein the material is a plastic or a glass.

11. A window, an optical filter, an ophthalmic lens or a sunglass lens made from a polymeric host material according to claim 9 or 10.

## Patentansprüche

1. Naphtho[1,2-b]pyran der allgemeinen Formel 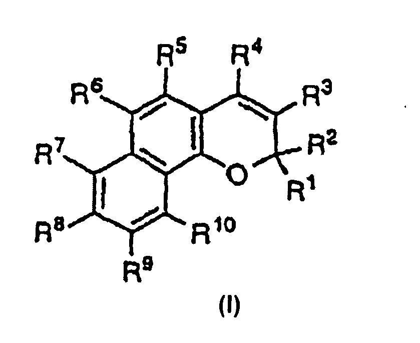 worin wenigstens eine von R¹ und R² eine 4-Aminoarylgruppe ist, worin die Arylgruppe Phenyl oder Naphthyl ist, und worin die Aminogruppe, die einen Teil der 4-Aminoarylgruppe für R¹ und R² bildet, C₁-C₅-Alkylamino, C₁-C₅-Dialkylamino, Arylamino, Diarylamino, Aryl-C₁-C₅-alkylamino oder eine cyclische Aminogruppe ist, worin die 4-Aminoarylgruppe zusätzlich zu der spezifizierten Aminofunktion optional und in jeder der verbleibenden Stellungen mit Wasserstoff, C₁-C₅-Alkyl, C₁-C₅-Halogenalkyl, C₁-C₅-Alkoxy, C₁-C₅-Alkoxy(C₁-C₅)alkyl, C₁-C₅-Hydroxyalkyl, C₁-C₅-Aminoalkyl, Halogen, Alkyl-C₁-C₅-amino, Dialkyl-C₁-C₅-amino, Arylamino, Diarylamino oder einer cyclischen Aminogruppe weiter substituiert sein kann; und worin, wo R¹ oder R² keine 4-Aminoarylgruppe ist, sie ausgewählt ist aus Phenyl, substituiertem Phenyl, Naphthyl, substituiertem Naphthyl, Thienyl, Benzo[b]-thienyl, Furyl, Benzo[b]furyl, Pyrryl oder Indolyl;
R⁵ ausgewählt ist aus linearem oder verzweigtem C₁-C₁₀-Alkyl, bis zu C₂₀-Cycloalkyl, bis zu C₂₀-Bicycloalkyl, bis zu C₂₀-Polycycloalkyl, linearem oder verzweigtem bis zu C₁₀-Alkenyl, bis zu C₁₀-Alkinyl, linearem oder verzweigtem C₁-C₁₀-Alkoxy, linearem oder verzweigtem C₁-C₁₀-Alkylthio, linearem oder verzweigtem C₁-C₁₀-Alkoxy(linearem oder verzweigtem C₁-C₁₀-)alkyl, linearem oder verzweigtem C₁-C₁₀-Hydroxyalkyl, linearem oder verzweigtem C₁-C₁₀-Aminoalkyl, Phenyl, Halogen, Nitril, Nitro, linearem oder verzweigtem C₁-C₂₀-Alkoxycarbonyl, Formyl, Acetyl, Aroyl oder die Alkenylfunktion ist: 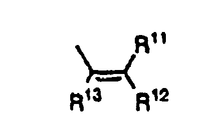 worin R¹¹ und/oder R¹² und/oder R¹³ Wasserstoff ist oder wie für R⁵ vorstehend definiert ist mit Ausnahme der Alkenylfunktion; und R³ und R⁴ Wasserstoff sind; R⁶ bis R¹⁰ jeweils Wasserstoff oder wie für R¹, R² oder R⁵ vorstehend definiert sind.

2. Naphtho[1,2-b]pyran gemäß Anspruch 1, worin die cyclische Aminogruppe Aziridino, Pyrrolidino, Piperidino, Morpholino, Thiomorpholino, Indolino, Piperazino, C₁-C₅-N-Alkylpiperazino oder N-Arylpiperazino ist.

3. Naphtho[1,2-b]pyran der allgemeinen Formel II, III oder IV: 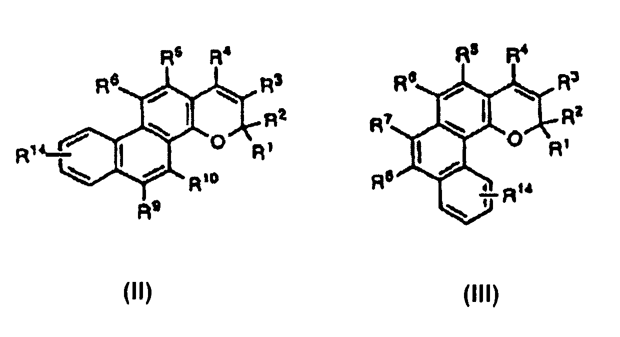 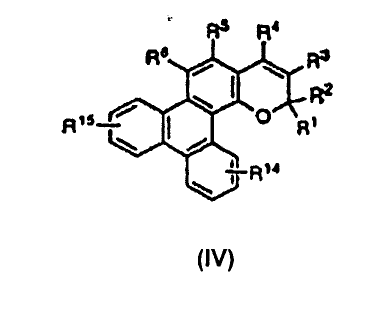 worin R¹ bis R¹³ einschließlich wie in Anspruch 1 oder 2 definiert sind, und worin R¹⁴ und R¹⁵ wie für R⁷ bis R¹⁰ definiert sind.

4. Naphtho[1,2-b]pyran der allgemeinen Formel V oder VI: 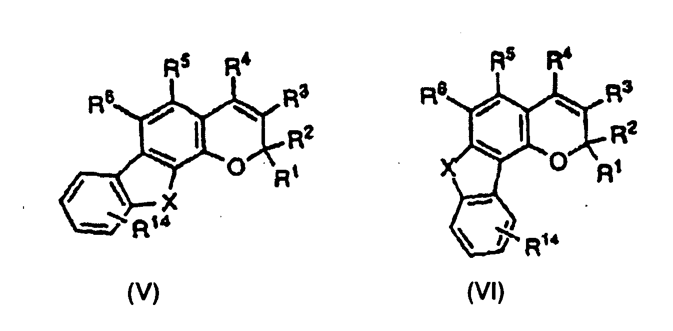 worin R¹ bis R¹⁴ einschließlich wie in Anspruch 3 definiert sind, X ausgewählt ist aus O, S, SO, SO₂, Se, NH, N-linearem oder verzweigtem C₁-C₁₀-Alkyl, N-Aryl, N-Heteroaryl, N-linearem oder verzweigtem C₁-C₁₀-Halogenalkyl, N-linearem oder verzweigtem C₁-C₁₀-Perhalogenalkyl, N-linearem oder verzweigtem C₁-C₁₀-Hydroxyalkyl, N-linearem oder verzweigtem C₁-C₁₀-Alkoxyalkyl, Benzyl, substituiertem Benzyl und Tosyl.

5. Naphtho[1,2-b]pyran gemäß einem der vorhergehenden Ansprüche, worin R¹ 4-Morpholinophenyl, 4-Piperidinophenyl, 4-Dimethylaminophenyl oder 4-Pyrrolidinophenyl ist und R⁵ Methoxycarbonyl ist.

6. Naphtho[1,2-b]pyran gemäß einem der Ansprüche 1 bis 5, worin R¹ und R² jeweils 4-Pyrrolidinophenyl sind und R⁵ Methoxycarbonyl ist.

7. Naphtho[1,2-b]pyran gemäß einem der Ansprüche 1 bis 5, worin R¹ 4-Morpholinophenyl ist, R² 4-Methoxyphenyl ist und R⁵ Methoxycarbonyl ist.

8. Naphtho[1,2-b]pyran gemäß einem der Ansprüche 1 bis 5, worin R¹ 4-Morpholinophenyl ist, R² 2-Thienyl ist und R⁵ Methoxycarbonyl ist.

9. Polymeres Wirtsmaterial, enthaltend ein Naphtho[1,2-b]pyran gemäß einem der vorhergehenden Ansprüche.

10. Polymeres Wirtsmaterial gemäß Anspruch 9, worin das Material ein Kunststoff oder ein Glas ist.

11. Fenster, optisches Filter, ophthalmische Linse oder Sonnenbrillenlinse, hergestellt aus einem polymeren Wirtsmaterial gemäß Anspruch 9 oder 10.

## Revendications

1. Naphto[1,2-b]pyrane de formule générale (I) 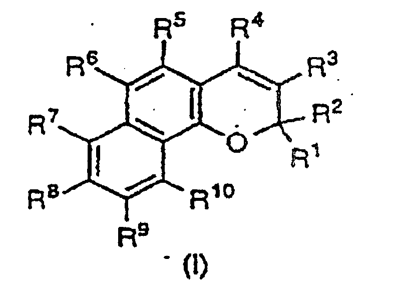 Dans laquelle au moins un des radicaux R¹ et R² est un groupe 4-aminoaryle, où le groupe aryle est un groupe phényle ou naphtyle, et dans laquelle le groupe amino qui fait partie du groupe 4-aminoaryle pour R¹ et R² est un groupe alkylamino en C₁-C₅, dialkylamino en C₁-C₅, arylamino, diarylamino, aryl-alkyl(en C₁-C₅)amino ou amino cyclique, où le groupe 4-aminoaryle peut éventuellement être encore substitué, en plus de la fonction amino précisée, et sur toutes les positions restantes, par un atome d'hydrogène, un groupe alkyle en C₁-C₅, halogénoalkyle en C₁-C₅, alcoxy en C₁-C₅, alcoxy(en C₁-C₅)alkyle (en C₁-C₅), hydroxyalkyle en C₁-C₅, aminoalkyle en C₁-C₅, un halogène, alkyl(en C₁-C₅)amino, dialkyl(en C₁-C₅)amino, arylamino, diarylamino ou amino cyclique ; et dans laquelle, lorsque R¹ ou R² n'est pas un groupe 4-aminoaryle, il est choisi parmi les groupes phényle, phényle substitué, naphtyle, naphtyle substitué, thiényle, benzo[b]thiényle, furyle, benzo[b]furyle, pyrryle ou indolyle ;
R⁵ est choisi parmi un groupe alkyle en C₁-C₁₀ linéaire ou ramifié, cycloalkyle jusqu'à C₂₀, bicycloalkyle jusqu'à C₂₀, polycycloalkyle jusqu'à C₂₀, alcényle jusqu'à C₁₀ linéaire ou ramifié, alcynyle jusqu'à C₁₀, alcoxy en C₁-C₁₀ linéaire ou ramifié, alkylthio en C₁-C₁₀ linéaire ou ramifié, alcoxy(en C₁-C₁₀ linéaire ou ramifié)-alkoxy (en C₁-C₁₀ linéaire ou ramifié), hydroxyalkyle en C₁-C₁₀ linéaire ou ramifié, aminoalkyle en C₁-C₁₀ linéaire ou ramifié, phényle, un halogène, nitrile, nitro, alcoxy(en C₁-C₂₀ linéaire ou ramifié)carbonyle, formyle, acétyle, aroyle, ou est la fonction alcényle : 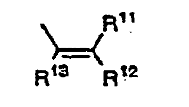 dans laquelle R¹¹ et/ou R¹² et/ou R¹³ est un atome d'hydrogène ou est défini comme pour R⁵ ci-dessus, à l'exclusion de la fonction alcényle ; et R³ et R⁴ sont des atomes d'hydrogène ; R⁶-R¹⁰ sont chacun des atomes d'hydrogène ou sont définis comme pour R¹, R² ou R⁵ ci-dessus.

2. Naphto[1,2-b]pyrane selon la revendication 1, dans lequel le groupe amino cyclique est un groupe aziridino, pyrrolidino, pipéridino, morpholino, thiomorpholino, indolino, pipérazino, N-alkyl(cn C₁-C₅)pipérazino ou N-arylpipérazino.

3. Naphto[1,2-b]pyrane de formule générale II, III ou IV : 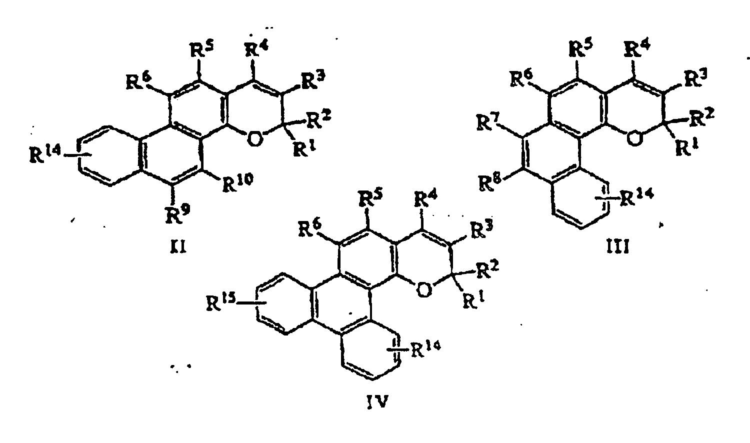 formules dans lesquelles R¹ à R¹³, bornes comprises, sont tels que définis dans la revendication 1 ou 2 et dans laquelle R¹⁴ et R¹⁵ sont définis comme pour R⁷-R¹⁰.

4. Naphto[1,2-b]pyrane de formule générale V ou VI : 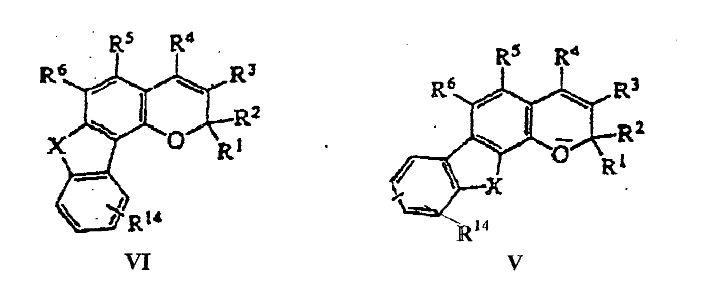 dans lesquelles R¹ à R¹⁴, bornes comprises, sont tels que définis dans la revendication 3, X est choisi parmi O, S, SO, SO₂, Se, NH, N-alkyle en C₁-C₁₀ linéaire ou ramifié, N-aryle, N-hétéroaryle, N-halogénoalkyle en C₁-C₁₀ linéaire ou ramifié, N-perhalogénoalkyle en C₁-C₁₀ linéaire ou ramifié, N-hydroxyalkyle en C₁-C₁₀ linéaire ou ramifié, N-alcoxyalkyle en C₁-C₁₀ linéaire ou ramifié, benzyle, benzyle substitué et tosyle.

5. Naphto[1,2-b]pyrane selon l'une quelconque des revendications précédentes, dans lequel R¹ est un groupe 4-morpholinophényle, 4-pipéridinophényle, 4-diméthylaminophényle ou 4-pyrrolidinophényle et R⁵ est un groupe méthoxycarbonyle.

6. Naphto[1,2-b]pyrane selon l'une quelconque des revendications 1 à 5, dans lequel R¹ et R² sont chacun un groupe 4-pyrrolidinophényle et R⁵ est un groupe méthoxycarbonyle.

7. Naphto[1,2-b]pyrane selon l'une quelconque des revendications 1 à 5, dans lequel R¹ est un groupe 4-morpholinophényle, R² est 4-méthoxyphenyle et R⁵ est un groupe méthoxycarbonyle.

8. Naphto[1,2-b]pyrane selon l'une quelconque des revendications 1 à 5, dans lequel R¹ est un groupe 4-morpholinophényle, R² est un groupe 2-thiényle et R⁵ est un groupe méthoxycarbonyle.

9. Matériau hôte polymérique contenant un naphta[1,2-b]pyrane selon l'une quelconque des revendications précédentes.

10. Matériau hôte polymérique selon la revendication 9, dans lequel le matériau est une matière plastique ou un verre.

11. Fenêtre, filtre optique, lentille ophtalmique ou lentille de lunettes de soleil, fabriqués à partir d'un matériau hôte polymérique selon la revendication 9 ou 10.
